# FASCICULE DE BREVET EUROPEEN

(11) **EP 0 734 730 B1**
(45) Date de publication et mention de la délivrance du brevet: **05.09.2001**
(21) Numéro de dépôt: 96400459.2
(22) Date de dépôt: 04.03.1996
(51) Int. Cl.: A61K 38/22, A61K 39/395, A61K 7/02

(54) **Utilisation d'un antagoniste de CGRP pour le traitement des prurits et des dysesthésies oculaires ou palpébraux**
Verwendung von einem CGRP Antagonist für die Behandlung von Augen oder Augenlidpruritus und Dysesthesien
Use of a CGRP antagonist for the treatment of ocular or eyelid pruritis and disesthesies

(30) Priorité: 28.03.1995 FR 9503629
(43) Date de publication de la demande: 02.10.1996
(73) Titulaire: L'OREAL, 75008 Paris (FR)
(72) Inventeur: de Lacharriere, Olivier, F-75015 Paris (FR); Breton, Lionel, F-78000 Versailles (FR)
(74) Mandataire: Lhoste, Catherine

(56) Documents cités:
- WO-A-93/21911
- NEUROSCIENCE, vol. 48, no. 4, Juin 1992, pages 963-968, XP000577183 T.L. BUCKLEY ET AL.: "THE PARTIAL INHIBITION OF INFLAMMATORY RESPONSES INDUCED BY A CAPSAICIN USING THE FAB FRAGMENT OF A SELECTIVE CALCITONIN GENE-RELATED PEPTIDE ANTISERUM IN RABBIT SKIN"
- BRITISH JOURNAL OF PHARMACOLOGY, vol. 110, no. 2, 1993, pages 772-776, XP000563605 K. JANE ESCOTT ET AL.: "EFFECT OF CALCITONIN GENE-RELATED PEPTIDE ANTAGONIST (CGRP 8-37) ON SKIN VASODILATATION AND OEDEMA INDUCED BY STIMULATION OF THE RAT SAPHENOUS NERVE"
- BRITISH JOURNAL OF PHARMACOLOGY, vol. 104, no. 3, Novembre 1991, pages 738-742, XP000563606 S.R. HUGHES ET AL.: "A CALCITONIN GENE-RELATED PEPTIDE (CGRP) ANTAGONIST (CGRP 8-37) INHIBITS MICROVASCULAR REPONSES INDUCED BY CGRP AND CAPSAICIN IN SKIN."
- NEUROSCIENCE LETTERS, vol. 102, no. 2.3, 31 Juillet 1989, pages 257-260, XP000578094 S.M. LOUIS ET AL.: "ANTIBODIES TO CALCITONIN-GENE RELATED PEPTIDE REDUCE INFLAMMATION INDUCED BY TOPICAL MUSTARD OIL BUT NOT THAT DUE TO CARRAGEENIN IN THE RAT"
- : "", EUROP. J. OF PHARMACOLOGY, , , Vol. 192, no. 1991, pages 85 à 88
- : "", EUROPEAN J. OF PHARMACOLOGY , , 1994, Vol. 265, no. , pages 53 à 59
- : "Le Petit Larousse", 1995, LAROUSSE, PARIS

## Description

La présente invention se rapporte à l'utilisation d'un antagoniste de CGRP (peptide dérivé du gène de la calcitonine : Calcitonin Gene Related Peptide en terminologie anglosaxonne) pour la préparation d'une composition pharmaceutique pour traiter notamment par voie topique les prurits et/ou algies et/ou dysesthésies oculaires et/ou palpébraux. Elle se rapporte aussi à l'utilisation d'un antagoniste de CGRP dans une composition cosmétique destinée au soin ou au maquillage des yeux ou des paupières ainsi qu'à un procédé de soin et/ou de maquillage des yeux sensibles.

Certains patients souffrent d'algies oculaires et/ou palpébrales à la suite d'opérations ou de coups reçus sur l'oeil. Par ailleurs, certaines personnes présentent trés fréquemment sans qu'on n'en connaisse la cause précise des sensations de démangeaisons ou prurit et des sensations dysesthésiques au niveau des yeux et des paupières. Il peut s'agir également de prurits ou sensations dysesthésies d'origine allergique.

Par sensations dysesthésiques, on entend des sensations de brûlures ou d'échauffement, de picotements, de fourmillements, d'inconforts et de tiraillements. Ces sensations peuvent s'associer à des rougeurs.

L'ensemble de ces signes ophtalmiques peuvent, en outre, s'associer à une rosacée et éventuellement à une conjonctivite.

Parmi les facteurs déclenchants les crises prurigineuses ou dysesthésiques ophtalmiques ou palpébrales, on peut citer les variations de température rapide, la chaleur et notamment l'exposition aux ultraviolets ou aux infrarouges, l'humidité relative basse, l'exposition aux vents violents ou aux courants d'air (soufflerie, air conditionné), l'application de tensioactifs, l'exposition à des vapeurs toxiques ou irritantes (solvants) ou à des poussières, les gouttes ou topiques ophtalmologiques irritants, les topiques palpébraux dermatologiques ou cosmétiques irritants (alpha-hydroxy-acides, rétinoïdes), ou l'utilisation de certains cosmétiques même lorsque ceux-ci ne sont pas connus comme particulièrement irritants.

Comme autres facteurs déclenchants les crises prurigineuses ou dysesthésiques oculaires ou palpébrales, il faut, en outre, inclure les allergènes comme notamment le pollen, les poils d'animaux, les acariens, les moisissures.

Jusqu'alors, le mécanisme pathologique de ces signes était très mal connu et les dysesthésies oculaires et/ou palpébrales étaient traitées par des corticoïdes et également des antiseptiques locaux en pommade ophtalmique ou en gouttes.

Les corticoïdes sont relativement efficaces pour calmer les symptômes ci-dessus, mais malheureusement, ils présentent des effets secondaires souvent très pénalisants comme des atrophies. De plus, ils sensibilisent aux infections mycosiques ou bactériennes et leur cinétique d'action est souvent lente (plusieurs minutes à quelques heures). Par ailleurs, leur utilisation chronique peut conduire à une pharmacodépendance.

Il subsiste donc le besoin d'une composition pour le traitement des prurits, des algies et des dysesthésies oculaires et palpébraux ne présentant pas les inconvénients ci-dessus.

La présente invention a justement pour objet l'utilisation d'un ou plusieurs antagonistes de CGRP pour traiter ces affections.

Le CGRP est un élément chimique polypeptidique élaboré et libéré par une terminaison nerveuse. Le CGRP intervient notamment dans des maladies respiratoires et inflammatoires, dans des maladies allergiques et dans certaines maladies dermatologiques telles que l'eczéma ou le prurigo.

La demanderesse a maintenant découvert qu'il était possible de traiter les prurits oculaires et/ou palpébraux et/ou les algies oculaires et/ou palpébrales et/ou les dysesthésies oculaires et/ou palpébrales en empêchant la synthèse et/ou la libération et/ou la fixation du CGRP.

Aussi, la présente invention a pour objet l'utilisation d'au moins un antagoniste de CGRP pour la préparation d'une composition pharmaceutique ou dermatologique pour traiter les prurits oculaires et/ou palpébraux et/ou les algies oculaires et/ou palpébrales et/ou les dysesthésies oculaires et/ou palpébrales.

Certes, il est connu des documents Neuroscience, vol 48, n°4, pp. 963-968 (1992) de T.L. Buckley et al., Br. J. Pharmacol. (1991), vol. 104 pp. 738-742 de S.R. Hughes et al., et Br. J. Pharmacol (1993), vol. 110, pp. 772-776 de K.J. Escott et al. que l'injection d'antagoniste de CGRP dans une veine ou la peau d'un animal inhibe les effets de la capsaïcine injectée ou appliquée topiquement à cet animal. Cependant, ces documents n'enseignent pas ni suggèrent que l'on puisse traiter les yeux sensibles avec une composition contenant un antagoniste de CGRP éventuellement associé à un antagoniste de neuropeptides.

Par ailleurs, il est connu du document WO-A-93/21911 d'utiliser des antagonistes de CGRP dans des compositions ophtalmiques pour empêcher la diminution de la pupille lors d'opération de l'oeil ou d'uvéite et non pour traiter les yeux sensibles et les signes cliniques des yeux sensibles.

L'application de compositions contenant un ou plusieurs antagonistes de CGRP sur les yeux ou les paupières permet d'obtenir une nette diminution voire une disparition complète des algies, sensations dysesthésiques et prurits ophtalmiques ; on constate très rapidement, et en tout état de cause beaucoup plus rapidement qu'avec les corticoïdes, un effet calmant et apaisant, préventif et curatif sur les yeux et les paupières. En outre, on ne note aucune pharmacodépendance.

Grâce à ces antagonistes de CGRP, il est en outre possible de concevoir des compositions cosmétiques pour yeux sensibles et en particulier des lotions démaquillantes ou de nettoyage des yeux, des produits de maquillage pour yeux sensibles et notamment des fards à paupières, des mascaras, des crayons ou des eye-liners pour yeux sensibles.

Aussi, l'invention à encore pour objet l'utilisation d'au moins un antagoniste de CGRP dans une composition cosmétique contenant un milieu cosmétiquement acceptable, destinée aux yeux sensibles.

L'invention a encore pour objet un procédé de soin ou de maquillage des yeux sensibles, consistant à appliquer sur les paupières, les cils et/ou sous les yeux une composition cosmétique contenant au moins un antagoniste de CGRP, dans un milieu cosmétiquement acceptable.

L'invention a encore pour objet une composition cosmétique, dermatologique et/ou pharmaceutique pour yeux sensibles, caractérisée en ce qu'elle contient au moins un antagoniste de CGRP, dans un milieu cosmétiquement, pharmaceutiquement ou dermatologiquement acceptable.

La composition de l'invention contient un milieu cosmétiquement, pharmaceutiquement ou dermatologiquement acceptable, c'est-à-dire un milieu compatible avec la peau, et les yeux. La composition contenant le ou les antagonistes de CGRP est appliquée notamment par voie topique. Elle peut également être ingérée ou injectée.

La demanderesse définit un antagoniste de CGRP comme toute molécule d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment à la caractéristique suivante : avoir une activité pharmacologique antagoniste du CGRP, c'est-à-dire induire une réponse pharmacologique cohérente notamment dans l'un des tests suivants :
- la substance antagoniste doit diminuer la vasodilatation induite par la capsaïcine et/ou
- la substance antagoniste doit provoquer une inhibition de la libération de CGRP par les fibres nerveuses sensitives et/ou
- la substance antagoniste doit diminuer une inhibition de la contraction du muscle lisse du canal déférent induite par le CGRP.

En outre, l'antagoniste peut avoir une affinité pour les récepteurs au CGRP.

Personne jusqu'à ce jour n'avait établi un lien entre le CGRP et les yeux sensibles.

Comme antagoniste de CGRP utilisable dans l'invention, on peut citer par exemple le CGRP 8-37 et les anticorps anti-CGRP.

Dans les compositions selon l'invention, l'antagoniste de CGRP est utilisé de préférence en une quantité allant de 0,000001 à 10 % en poids par rapport au poids total de la composition, et en particulier en une quantité allant de 0,0001 à 5 % en poids par rapport au poids total de la composition.

De façon avantageuse, on peut associer à l'antagoniste de CGRP un ou plusieurs antagonistes d'un autre neuropeptide comme des antagonistes de substance P et/ou un ou plusieurs antagonistes de médiateur de l'inflammation comme des antagonistes d'histamine, des antagonistes d'interleukine 1 (IL1), et des antagonistes de Tumor Necrosis Factor alpha (TNF alpha).

De préférence les antagonistes de substance P sont des antagonistes réceptoriels.

Les antagonistes de substance P utilisables dans l'invention sont notamment ceux décrits dans la demande de brevet 94/05537 déposée le 05 mai 1994 au nom de la demanderesse. Comme antagoniste de substance P utilisable dans l'invention, on peut citer par exemple le sendide et le spantide II.

A titre d'exemple, les antagonistes de substance P et les antagonistes de médiateur de l'inflammation peuvent être utilisés en une quantité représentant de 0,000001 à 10 % du poids total de la composition et mieux de 0,0001 à 5 %.

Comme antagonistes de médiateur de l'inflammation utilisables dans l'invention, on peut citer les dérivés de la diéthylène diamine telle que cinnarizine, cyclizine ; les dérivés de l'aminopropane (dexchlorophéniramine, tripolidine) ; des dérivés de phénothiazine (alimémazine, prométhazine) ; l'auranofine ; la lisophyline ; l'A802715 ; la sulfasalazine ; la cétirizine HCI ; la loratidine ; l'esbatine ; la sétastine Hcl.

Les compositions selon l'invention peuvent se présenter sous toutes les formes galéniques normalement utilisées pour une application topique ; la composition peut se présenter notamment sous forme de solutions aqueuses, hydroalcooliques ou huileuses, ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel aqueux ou anhydre, de microémulsions, de microcapsules, de microparticules, de dispersions vésiculaires de type ionique et/ou non ionique, de poudres compactées ou coulées. Ces compositions sont préparées selon les méthodes usuelles.

Pour une application topique à visée thérapeutique, les compositions se présentent notamment sous forme de gel, crème, pommade pour le traitement des prurits et/ou algies et/ou dysesthésies palpébraux et sous forme de collyre ou de solution de lavage oculaire oculaire pour le traitement des prurits et/ou algies et/ou dysesthésies oculaires.

Pour une application cosmétique, les compositions peuvent notamment constituées des crèmes de soin ou de protection pour yeux sensibles, des laits ou lotions de nettoyage ou de démaquillage des yeux sensibles, des produits de maquillage des yeux notamment sensibles comme des crayons, des mascaras, des eye-liners, des fards à paupières.

Les compositions injectables peuvent se présenter sous forme d'une lotion aqueuse, huileuse ou sous forme de sérum.

Les compositions utilisées par voie orale peuvent se présenter sous forme de capsules, de gélules, de sirops ou de comprimés.

Les quantités des différents constituants des compositions selon l'invention sont celles classiquement utilisées dans les domaines considérés.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines considérés, tels que les gélifiants hydrophiles ou lipophiles, les actifs hydrophiles ou lipophiles, les conservateurs, les antioxydants, les solvants, les parfums, les charges, les filtres solaires, les absorbeurs d'odeur, des pigments et les matières colorantes. Les quantités de ces différents adjuvants sont celles classiquement utilisées dans les domaines considérés, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser des alcools gras, des acides gras (acide stéarique) ou encore des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines, l'amidon, les extraits végétaux, notamment d'Aloe Vera.

Comme actifs lipophiles, on peut utiliser le tocophérol (vitamine E) et ses dérivés, le rétinol (vitamine A) et ses dérivés, les acides gras essentiels, les céramides, les huiles essentielles.

On peut également associer les antagonistes de CGRP à des agents actifs, notamment des cicatrisants (par exemple vitamine B₁₂), des antiseptiques (par exemple acide borique), des antiallergiques (par exemple chromoglycate de sodium), des antiviraux (par exemple acyclovir), des anesthésiques (par exemple chlorhydrate de lidocaïne et dérivés) et des anti-inflammatoires non stéroïdiens (par exemple indométhacine).

Les exemples suivants illustrent l'invention. Dans ces exemples, les proportions indiquées sont des pourcentages en poids.

### Exemple 1: Collyre

| ***Collyre*** | |
|---|---|
| CGRP 8-37 | qsp 0,5 % |
| Excipient : | 100 % |
| Chlorure de sodium | |
| Borate de sodium | |
| Polysorbate 80 | |
| Acide borique | |
| Eau | |

### Exemple 2 : Pommade

| ***Pommade*** | |
|---|---|
| Anticorps Anti-CGRP | qsp 1 % |
| Excipient : | 100 % |
| Chlorure de benzalkonium | |
| Edetate de sodium | |
| D-mannitol | |
| Carbomer | |
| Soude | |
| Eau | |

### Exemple 3 Solution

| ***Solution*** | |
|---|---|
| CGRP 8-37 | 2 % |
| Excipient : | |
| Acide borique | 5 % |
| Chlorure de sodium | 0,3 % |
| Borate de phénylmercure | 0,5 % |
| Eau | qsp 100 % |

### Exemple 4 Pommade

Cet exemple se distingue de l'exemple 2 par l'adjonction de 0,1 % de sendide.

### Exemple 5 : Collyre

Cet exemple se différencie de l'exemple par l'addition de 0,3 % de loratidine.

## Revendications

1. Utilisation d'au moins un antagoniste de CGRP pour la préparation d'une composition pharmaceutique ou dermatologique, destinée à traiter les prurits oculaires et/ou palpébraux et/ou les algies oculaires et/ou palpébrales et/ou les dysesthésies oculaires ou palpébrales.

2. Utilisation d'au moins un antagoniste de CGRP dans une composition cosmétique contenant un milieu cosmétiquement acceptable, destinée aux yeux sensibles.

3. Utilisation selon la revendication 1 ou 2, **caractérisée en ce que** l'antagoniste de CGRP est une molécule qui diminue la vasodilatation induite par la capsaïcine.

4. Utilisation selon l'une quelconque des revendications précédentes, **caractérisée en ce que** l'antagoniste de CGRP est une molécule qui diminue une inhibition de la contraction du muscle lisse du canal déférent induite par le CGRP.

5. Utilisation selon l'une quelconque des revendications précédentes, **caractérisée en ce que** l'antagoniste de CGRP est une molécule choisie parmi le CGRP 8-37 et les anticorps anti-CGRP .

6. Utilisation selon rune quelconque des revendications précédentes, **caractérisée en ce que** l'antagoniste de CGRP est utilisé en une quantité allant de 0,000001 à 10 % en poids par rapport au poids total de la composition.

7. Utilisation selon l'une quelconque des revendications précédentes, **caractérisée en ce que** l'antagoniste de CGRP est utilisé en une quantité allant de 0,0001 à 5 % en poids par rapport au poids total de la composition.

8. Utilisation selon l'une quelconque des revendications précédentes, **caractérisée en ce que** la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les céramides, les huiles essentielles.

9. Utilisation selon l'une quelconque des revendications précédentes, **caractérisée en ce que** la composition contient en outre au moins un agent choisi parmi les agents cicatrisants, antiseptiques, antiallergiques, anesthésiques, antiviraux, anti-inflammatoires non stéroïdiens, les antagonistes de neuropeptides différents du CGRP, les antagonistes de médiateur de l'inflammation.

10. Utilisation selon l'une quelconque des revendications précédentes, **caractérisée en ce que** la composition se présente sous une forme appropriée à une application topique, injectable ou ingérable.

11. Utilisation selon l'une quelconque des revendications précédentes, **caractérisée en ce que** la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules, une poudre compactée ou coulée.

12. Utilisation selon l'une quelconque des revendications précédentes, **caractérisée en ce que** la composition est destinée au maquillage ou au démaquillage des yeux.

13. Utilisation selon l'une quelconque des revendications précédentes, **caractérisée en ce que** la composition contient en outre au moins un adjuvant choisi parmi les émulsionnants, les conservateurs, les antioxydants, les solvants, les gélifiants hydrophiles ou lipophiles, les actifs hydrophiles ou lipophiles, les parfums, les charges, les filtres solaires, les absorbeurs d'odeur, les pigments et les matières colorantes.

14. Composition cosmétique de soin ou de maquillage des yeux sensibles, **caractérisée en ce qu**'elle contient au moins un antagoniste de CGRP dans un milieu cosmétiquement, pharmaceutiquement ou dermatologiquement acceptable.

15. Composition selon la revendication 14, **caractérisée en ce que** l'antagoniste de CGRP est une molécule qui diminue la vasodilatation induite par la capsaïcine.

16. Composition selon la revendication 14 ou 15, **caractérisée en ce que** l'antagoniste de CGRP est une molécule qui diminue une inhibition de la contraction du muscle lisse du canal déférent induite par le CGRP.

17. Composition selon l'une quelconque des revendications 14 à 16, **caractérisée en ce que** l'antagoniste de CGRP est une molécule choisie parmi le CGRP 8-37 et les anticorps anti-CGRP.

18. Composition selon l'une quelconque des revendications 14 à 17, **caractérisée en ce que** l'antagoniste de CGRP est utilisé en une quantité allant de 0,000001 à 10 % en poids par rapport au poids total de la composition.

19. Composition selon l'une quelconque des revendications 14 à 18, **caractérisée en ce que** l'antagoniste de CGRP est utilisé en une quantité allant de 0,0001 à 5 % en poids par rapport au poids total de la composition.

20. Composition selon l'une quelconque des revendications 14 à 19, **caractérisée en ce que** la composition contient en outre au moins un agent choisi parmi les agents cicatrisants, antiseptiques, antiallergiques, anesthésiques, antiviraux, anti-inflammatoires non stéroïdiens, les antagonistes de neuropeptides différents du CGRP, les antagonistes de médiateur de l'inflammation.

21. Composition selon l'une quelconque des revendications 14 à 20, **caractérisée en ce que** la composition contient en outre au moins un agent choisi parmi les antagonistes de substance P.

22. Composition selon l'une quelconque des revendications 14 à 21, **caractérisée en ce que** la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules, une poudre compactée ou coulée.

23. Composition selon l'une quelconque des revendications 14 à 22, **caractérisée en ce que** la composition contient eh outre au moins un adjuvant choisi parmi les émulsionnants, les conservateurs, les antioxydants, les solvants, les gélifiants hydrophiles ou lipophiles, les actifs hydrophiles ou lipophiles, les parfums, les charges, les filtres solaires, les absorbeurs d'odeur, les pigments et les matières colorantes.

24. Composition selon l'une quelconque des revendications 14 à 23, **caractérisée en ce qu**'elle constitue un lait ou une lotion de nettoyage ou de démaquillage des yeux sensibles, un crayon, un mascara, un eye-liner, un fard à paupières pour yeux sensibles.

25. Procédé cosmétique de soin ou de maquillage des yeux sensibles, consistant à appliquer sur les paupières, les cils et/ou sous les yeux des personnes ayant les yeux sensibles une composition cosmétique contenant au moins un antagoniste de CGRP, dans un milieu cosmétiquement acceptable.

## Claims

1. Use of at least one CGRP antagonist for the preparation of a pharmaceutical or dermatological composition intended for treating ocular and/or palpebral pruritus and/or ocular and/or palpebral pains and/or ocular or palpebral dysaesthesias.

2. Use of at least one CGRP antagonist in a cosmetic composition containing a cosmetically acceptable medium, this composition being intended for sensitive eyes.

3. Use according to Claim 1 or 2, **characterized in that** the CGRP antagonist is a molecule which reduces the vasodilation induced by capsaicin.

4. Use according to any one of the preceding claims, **characterized in that** the CGRP antagonist is a molecule which reduces an inhibition of vas deferens smooth muscle contraction induced by CGRP.

5. Use according to any one of the preceding claims, **characterized in that** the CGRP antagonist is a molecule chosen from CGRP 8-37 and anti-CGRP antibodies.

6. Use according to any one of the preceding claims, **characterized in that** the CGRP antagonist is used in an amount ranging from 0.000001 to 10 % by weight relative to the total weight of the composition.

7. Use according to any one of the preceding claims, **characterized in that** the CGRP antagonist is used in an amount ranging from 0.0001 to 5 % by weight relative to the total weight of the composition.

8. Use according to any one of the preceding claims, **characterized in that** the composition also contains at least one active agent chosen from proteins or protein hydrolysates, amino acids, polyols, urea, sugars and sugar derivatives, vitamins, starch, plant extracts, ceramides and essential oils.

9. Use according to any one of the preceding claims, **characterized in that** the composition also contains at least one agent chosen from cicatrizing agents, antiseptics, antiallergic agents, anaesthetics, antiviral agents, non-steroidal anti-inflammatory agents, antagonists of neuropeptides other than CGRP and inflammation mediator antagonists.

10. Use according to any one of the preceding claims, **characterized in that** the composition is in a suitable form for topical, injectable or ingestible application.

11. Use according to any one of the preceding claims, **characterized in that** the composition is an aqueous, oily or aqueous-alcoholic solution, a water-in-oil emulsion, an oil-in-water emulsion, a microemulsion, an aqueous gel, an anhydrous gel, a serum, a dispersion of vesicles, microcapsules or microparticles, or a compacted or cast powder.

12. Use according to any one of the preceding claims, **characterized in that** the composition is intended for applying make-up to or removing make-up from the eyes.

13. Use according to any one of the preceding claims, **characterized in that** the composition also contains at least one adjuvant chosen from emulsifying agents, preserving agents, antioxidants, solvents, hydrophilic or lipophilic gelling agents, hydrophilic or lipophilic active agents, fragrances, fillers, sunscreens, odour absorbers, pigments and dyestuffs.

14. Cosmetic composition for making up or caring for sensitive eyes, **characterized in that** it contains at least one CGRP antagonist in a cosmetically, pharmaceutically or dermatologically acceptable medium.

15. Composition according to Claim 14, **characterized in that** the CGRP antagonist is a molecule which reduces the vasodilation induced by capsaicin.

16. Composition according to Claim 14 or 15, **characterized in that** the CGRP antagonist is a molecule which reduces an inhibition of vas deferens smooth muscle contraction induced by CGRP.

17. Composition according to any one of Claims 14 to 16, **characterized in that** the CGRP antagonist is a molecule chosen from CGRP 8-37 and anti-CGRP antibodies.

18. Composition according to any one of Claims 14 to 17, **characterized in that** the CGRP antagonist is used in an amount ranging from 0.000001 to 10 % by weight relative to the total weight of the composition.

19. Composition according to any one of Claims 14 to 18, **characterized in that** the CGRP antagonist is used in an amount ranging from 0.0001 to 5 % by weight relative to the total weight of the composition.

20. Composition according to any one of Claims 14 to 19, **characterized in that** the composition also contains at least one agent chosen from cicatrizing agents, antiseptics, antiallergic agents, anaesthetics, antiviral agents, non-steroidal anti-inflammatory agents, antagonists of neuropeptides other than CGRP and inflammation mediator antagonists.

21. Composition according to any one of Claims 14 to 20, **characterized in that** the composition also contains at least one agent chosen from substance P antagonists.

22. Composition according to any one of Claims 14 to 21, **characterized in that** the composition is an aqueous, oily or aqueous-alcoholic solution, a water-in-oil emulsion, an oil-in-water emulsion, a microemulsion, an aqueous gel, an anhydrous gel, a serum, a dispersion of vesicles, microcapsules or microparticles, or a compacted or cast powder.

23. Composition according to any one of Claims 14 to 22, **characterized in that** the composition also contains at least one adjuvant chosen from emulsifiers, preserving agents, antioxidants, solvents, hydrophilic or lipophilic gelling agents, hydrophilic or lipophilic active agents, fragrances, fillers, sunscreens, odour absorbers, pigments and dyestuffs.

24. Composition according to any one of Claims 14 to 23, **characterized in that** it constitutes a milk or a lotion for cleansing or for removing make-up from sensitive eyes, a pencil, a mascara, an eyeliner or an eyeshadow for sensitive eyes.

25. Cosmetic process for making up or caring for sensitive eyes, which consists in applying a cosmetic composition containing at least one CGRP antagonist, in a cosmetically acceptable medium, to the eyelids, the eyelashes and/or under the eyes of persons with sensitive eyes.

## Patentansprüche

1. Verwendung mindestens eines CGRP-Antagonisten zur Herstellung einer pharmazeutischen oder dermatologischen Zusammensetzung zur Behandlung von okularem und/oder palpebralem Juckreiz und/oder okularen und/oder palpebralen Schmerzen und/oder okularer oder palpebraler Dysästhesie.

2. Verwendung mindestens eines CGRP-Antagonisten in einer kosmetischen Zusammensetzung, die ein kosmetisch akzeptables Medium enthält und für empfindliche Augen vorgesehen ist.

3. Verwendung nach Anspruch 1 oder 2, **dadurch gekennzeichnet**, daß der CGRP-Antagonist ein Molekül ist, das die durch Capsaicin hervorgerufene Vasodilatation verringert.

4. Verwendung nach einem der vorhergehenden Ansprüche, **dadurch gekennzeichnet**, daß der CGRP-Antagonist ein Molekül ist, das eine Inhibierung der Kontraktion der glatten Muskulatur des Samenleiters, die durch CGRP hervorgerufen wird, vermindert.

5. Verwendung nach einem der vorhergehenden Ansprüche, **dadurch gekennzeichnet**, daß der CGRP-Antagonist unter CGRP 8-37 und anti-CGRP-Antikörpern ausgewählt ist.

6. Verwendung nach einem der vorhergehenden Ansprüche, **dadurch gekennzeichnet**, daß der CGRP-Antagonist in einem Mengenanteil von 0,000001 bis 10 Gew.-%, bezogen auf das Gesamtgewicht der Zusammensetzung, verwendet wird.

7. Verwendung nach einem der vorhergehenden Ansprüche, **dadurch gekennzeichnet**, daß der CGRP-Antagonist in einem Mengenanteil von 0,0001 bis 5 Gew.-%, bezogen auf das Gesamtgewicht der Zusammensetzung, verwendet wird.

8. Verwendung nach einem der vorhergehenden Ansprüche, **dadurch gekennzeichnet**, daß die Zusammensetzung ferner einen Wirkstoff enthält, der unter den Proteinen oder Proteinhydrolysaten, Aminosäuren, Polyolen, Harnstoff, Zuckern und Zuckerderivaten, Vitaminen, Stärke, Pflanzenextrakten, Ceramiden und etherischen Ölen ausgewählt ist.

9. Verwendung nach einem der vorhergehenden Ansprüche, **dadurch gekennzeichnet**, daß die Zusammensetzung ferner mindestens ein Mittel enthält, das unter den Vernarbungsmitteln, antiseptischen Mitteln, antiallergischen Mitteln, anästhesierenden Mitteln, antiviralen Mitteln, nicht steroidalen entzündungshemmenden Mitteln, Antagonisten von Neuropeptiden, die von CGRP verschieden sind, und Antagonisten von Entzündungsmediatoren ausgewählt ist.

10. Verwendung nach einem der vorhergehenden Ansprüche, **dadurch gekennzeichnet**, daß die Zusammensetzung in einer zur topischen Anwendung, zur Injektion oder zur Einnahme geeigneten Form vorliegt.

11. Verwendung nach einem der vorhergehenden Ansprüche, **dadurch gekennzeichnet**, daß die Zusammensetzung eine wässrige, ölige oder wässrig-alkoholische Lösung, Wasser-in-Öl-Emulsion, Öl-in-Wasser-Emulsion, Mikroemulsion, ein wässriges Gel, wasserfreies Gel, Serum, eine Vesikeldispersion, Mikrokapseldispersion, Mikropartikeldispersion oder ein gepreßtes oder gegossenes Pulver ist.

12. Verwendung nach einem der vorhergehenden Ansprüche, **dadurch gekennzeichnet**, daß die Zusammensetzung zum Schminken oder Abschminken der Augen vorgesehen ist.

13. Verwendung nach einem der vorhergehenden Ansprüche, **dadurch gekennzeichnet**, daß die Zusammensetzung auch mindestens einen Zusatzstoff enthält, der unter den Emulgatoren, Konservierungsmitteln, Antioxidantien, Lösungsmitteln, lipophilen oder hydrophilen Gelbildnern, lipophilen oder hydrophilen Wirkstoffen, Parfums, Füllstoffen, Sonnenschutzfiltern, Geruchsabsorbern, Pigmenten und Farbmitteln ausgewählt ist.

14. Kosmetische Zusammensetzung zur Pflege oder zum Schminken empfindlicher Augen, **dadurch gekennzeichnet**, daß sie in einem kosmetisch, pharmazeutisch oder dermatologisch akzeptablen Medium mindestens einen CGRP-Antagonisten enthält.

15. Zusammensetzung nach Anspruch 14, **dadurch gekennzeichnet**, daß der CGRP-Antagonist ein Molekül ist, das die durch Capsaicin hervorgerufene Vasodilatation verringert.

16. Zusammensetzung nach Anspruch 14 oder 15, **dadurch gekennzeichnet**, daß der CGRP-Antagonist ein Molekül ist, das eine Inhibierung der Kontraktion der glatten Muskulatur des Samenleiters, die durch CGRP hervorgerufen wird, vermindert.

17. Zusammensetzung nach einem der Ansprüche 14 bis 16, **dadurch gekennzeichnet**, daß der CGRP-Antagonist unter CGRP 8-37 und anti-CGRP-Antikörpern ausgewählt ist.

18. Zusammensetzung nach einem der Ansprüche 14 bis 17, **dadurch gekennzeichnet**, daß der CGRP-Antagonist in einem Mengenanteil von 0,000001 bis 10 Gew.-%, bezogen auf das Gesamtgewicht der Zusammensetzung, verwendet wird.

19. Zusammensetzung nach einem der Ansprüche 14 bis 18, **dadurch gekennzeichnet**, daß der CGRP-Antagonist in einem Mengenanteil von 0,0001 bis 5 Gew.-%, bezogen auf das Gesamtgewicht der Zusammensetzung, verwendet wird.

20. Zusammensetzung nach einem der Ansprüche 14 bis 19, **dadurch gekennzeichnet**, daß die Zusammensetzung ferner mindestens ein Mittel enthält, das unter den Vernarbungsmitteln, antiseptischen Mitteln, antiallergischen Mitteln, anästhesierenden Mitteln, antiviralen Mitteln, nicht steroidalen entzündungshemmenden Mitteln, Antagonisten von Neuropeptiden, die von CGRP verschieden sind, und Antagonisten von Entzündungsmediatoren ausgewählt ist.

21. Zusammensetzung nach einem der Ansprüche 14 bis 20, **dadurch gekennzeichnet**, daß die Zusammensetzung ferner mindestens ein Mittel enthält, das unter den Substanz P-Antagonisten ausgewählt ist.

22. Zusammensetzung nach einem der Ansprüche 14 bis 21, **dadurch gekennzeichnet**, daß die Zusammensetzung eine wässrige, ölige oder wässrig-alkoholische Lösung, Wasser-in-Öl-Emulsion, Öl-in-Wasser-Emulsion, Mikroemulsion, ein wässriges Gel, wasserfreies Gel, Serum, eine Vesikeldispersion, Mikrokapseldispersion, Mikropartikeldispersion oder ein gepreßtes oder gegossenes Pulver ist.

23. Zusammensetzung nach einem der Ansprüche 14 bis 22, **dadurch gekennzeichnet**, daß die Zusammensetzung auch mindestens einen Zusatzstoff enthält, der unter den Emulgatoren, Konservierungsmitteln, Antioxidantien, Lösungsmitteln, lipophilen oder hydrophilen Gelbildnern, lipophilen oder hydrophilen Wirkstoffen, Parfums, Füllstoffen, Sonnenschutzfiltern, Geruchsabsorbern, Pigmenten und Farbmitteln ausgewählt ist.

24. Zusammensetzung nach einem der Ansprüche 14 bis 23, **dadurch gekennzeichnet**, daß sie eine Milch oder Lotion zur Reinigung oder zum Abschminken von empfindlichen Augen, ein Konturenstift, Mascara, Eyeliner oder Lidschatten für empfindliche Augen ist.

25. Kosmetisches Verfahren zur Pflege oder zum Schminken empfindlicher Augen, das darin besteht, bei Personen mit empfindlichen Augen auf die Lider, die Wimpern und/oder unter die Augen eine kosmetische Zusammensetzung aufzubringen, die in einem kosmetisch akzeptablen Medium mindestens einen CGRP-Antagonisten enthält.
